# FASCICULE DE BREVET EUROPEEN

(11) **EP 3 708 127 B1**
(45) Date de publication et mention de la délivrance du brevet: **20.08.2025**
(21) Numéro de dépôt: 20162822.9
(22) Date de dépôt: 12.03.2020
(51) Int. Cl.: A61F 2/36, A61F 2/40

(54) **TIGE MEDULLAIRE D'ENDOPROTHÈSE D'ARTICULATION**
MEDULLÄRER STAB EINER GELENKENDOPROTHESE
MEDULLARY ROD OF JOINT ENDOPROSTHESIS

(30) Priorité: 12.03.2019 FR 1902534
(43) Date de publication de la demande: 16.09.2020
(73) Titulaire: FX Shoulder Solutions, 01440 Viria (FR)
(72) Inventeur: JEROSCH, Jörg, 40667 Meerbusch (DE); NOURISSAT, Geoffroy, 94300 Vincennes (FR); BURKHEAD JR., Wayne Buz, Dallas TX 75229 (US); COSTOUROS, John, Hillsborough CA 94010 (US); MOEN, Todd Christopher, Dallas TX 75203 (US)
(74) Mandataire: LLR

(56) Documents cités:
- BE-A6- 1 002 354
- US-A- 5 032 134
- US-A1- 2011 082 551
- US-A1- 2012 064 288

## Description

La présente invention concerne une tige médullaire d'une endoprothèse d'articulation, destinée à être mise en place dans le canal médullaire d'un os long que comprend l'articulation concernée.

Il est bien connu de restaurer une articulation incluant un os long au moyen d'une endoprothèse d'articulation. Cette endoprothèse comprend un implant médullaire effilé destiné à être mis en place sur l'os long par son insertion dans le canal médullaire de cet os, dument réséqué, et un implant conjugué destiné à être mis en place sur l'autre os constituant l'articulation. En particulier, une endoprothèse d'épaule comprend un implant huméral, destiné à être en place sur l'os long que constitue l'humérus, et un implant de glène destiné à être mis en place sur l'omoplate. L'implant huméral comprend une tige médullaire effilée, destinée à être insérée dans le canal médullaire de l'humérus, après fraisage et alésage de la partie proximale de cet os formant le canal médullaire ; l'implant huméral comprend également une partie proximale destinée à être montée sur cette tige effilée, qui forme une surface articulaire. Cette partie proximale peut comprendre, dans le cas d'une endoprothèse d'épaule dite "anatomique" une tête prothétique formant une surface articulaire convexe, apte à coopérer avec une surface articulaire conjuguée concave ; dans le cas d'une endoprothèse d'épaule dite "inversée", la partie proximale comprend une cavité formant une surface articulaire concave, qui est destinée à coopérer avec une surface articulaire convexe conjuguée formée par l'implant de glène.

Sur une endoprothèse de hanche, l'implant médullaire est une tige fémorale et l'implant conjugué est un cotyle mis en place au niveau de la cavité cotyloïdienne du bassin du patient.

Une tige médullaire classique est formée par une pièce pleine et massive, faite en un matériau métallique, en général de l'alliage de titane. Une telle tige est apte à résister aux efforts nombreux et répétés subis par l'endoprothèse du fait des mouvements du patient.

Cependant, il apparaît que les cas de désolidarisation d'une tige médullaire par rapport à l'os recevant cette tige sont fréquents. Il est connu que cette désolidarisation est causée par le fait que la tige a une rigidité importante, nettement supérieure à celle de l'os, lequel est un matériau vivant qui est en perpétuelle régénération. De plus, il apparaît que l'os ne se régénère pas au niveau de certaines parties osseuses ayant reçu l'endoprothèse du fait que ces parties osseuses ne sont plus sollicitées par les efforts exercés sur l'endoprothèse. Une lyse osseuse peut alors être observée au long de l'endoprothèse, ainsi qu'une fonte des tubérosités assurant l'ancrage de cette endoprothèse.

L'invention a pour objectif de remédier à ce problème.

Le document US 2012/0064288 A1 divulgue une tige médullaire selon le préambule de la revendication 1.

À cet effet, l'invention a pour objet une tige médullaire selon la revendication 1.

Il sera compris que les bords interne et externe de l'os long en question sont les portions longitudinales de cet os qui se trouvent respectivement, lorsque l'os est vu dans le plan frontal, sur le côté interne du patient et sur le côté externe de ce patient.

La tige médullaire selon l'invention ne présente ainsi pas une structure monolytique, pleine et massive, comme cela est le cas d'une tige médullaire classique en matériau métallique, mais présente une structure à paroi d'enveloppe et à pluralité de cloisons à l'intérieur de cette paroi d'enveloppe, espacées les unes des autres et formant corps avec ladite paroi d'enveloppe. Cette structure s'avère particulièrement intéressante en ce sens qu'elle est apte à supporter la charge s'exerçant sur la tige médullaire, du fait de la pluralité de cloisons et du fait que ces cloisons forment corps avec la paroi d'enveloppe, tout en présentant une légère capacité de déformation proche de celle d'un os.

La paroi d'enveloppe peut avoir une épaisseur allant de 0,5 à 2 mm environ. Cette épaisseur est variable selon la taille de la tige médullaire, une telle tige étant en différentes tailles adaptées aux différentes tailles de patients. Elle pourra être de l'ordre de 0,5 mm pour une tige de plus petite taille dans une gamme de tiges médullaires, et de l'ordre de 2 mm pour une tige de plus grande taille dans cette gamme.

Chaque cloison peut avoir une épaisseur allant de 0,5 à 1,5 mm environ. L'espace entre les cloisons peut aller de 1 à 2 mm environ. Ces dimensions sont également variables selon la taille de la tige médullaire, une telle tige étant en différentes tailles adaptées aux différentes tailles de patients.

Le nombre de cloisons peut aller de quatre à six cloisons sur une tige médullaire destinée à un patient de petite taille et de six à dix cloisons sur une tige médullaire destinée à patient de grande taille.

Les cloisons peuvent être planes ou présenter un profil ondulé.

La tige médullaire peut comprendre également une pluralité de deuxièmes cloisons, généralement parallèles entre elles, ayant une orientation différente de celle desdites premières cloisons et venant ainsi croiser ces premières cloisons, chacune de ces deuxièmes cloisons formant corps avec la paroi d'enveloppe de la tige médullaire et formant corps avec lesdites premières cloisons.

L'ensemble des premières et deuxièmes cloisons présente ainsi, vu en section transversale, une structure en croisillons.

Lesdites premières cloisons peuvent avoir une orientation purement interne-externe, c'est-à-dire s'étendre parallèlement à un plan frontal du patient ; ces premières cloisons, et des deuxièmes cloisons telles que précitées, peuvent également s'étendre selon des angles non droits par rapport à ce plan frontal ; les angles formés par les premières cloisons par rapport à ce plan frontal peuvent être égaux aux angles formés par les deuxièmes cloisons par rapport à ce même plan frontal, de sorte que les premières et deuxièmes cloisons soient disposées en croix de saint André par rapport à ce plan frontal.

Lesdites deuxièmes cloisons permettent l'optimisation de la résistance de l'endoprothèse aux contraintes répétées qu'une telle endoprothèse est destinée à subir, tout en ayant une légère capacité de déformation correspondant au module de Young de l'os, donc limitant le risque de désolidarisation de l'endoprothèse d'avec cet os.

Les alvéoles délimitées par lesdites premières et deuxièmes cloisons peuvent présenter une section transversale sensiblement circulaire, octogonale, triangulaire ou carrée, selon la forme et/ou l'orientation desdites cloisons les unes par rapport aux autres.

Alternativement, lesdites premières cloisons et deuxièmes cloisons délimitent entre elles des alvéoles ayant une structure en nid d'abeilles.

Une telle tige médullaire s'avère présenter des performances optimales pour l'obtention du résultat recherché, à savoir le fait de fournir une endoprothèse à la fois résistante et présentant un léger degré de déformabilité élastique, proche de celui d'un os.

La tige médullaire présente une structure interne longitudinale, à section transversale pleine, formant corps avec la paroi d'enveloppe et lesdites premières cloisons et deuxièmes cloisons lorsque ces deuxièmes cloisons sont présentes.

Cette structure permet un renforcement longitudinal de la tige.

Cette structure interne est sous la forme d'un barreau interne central s'étendant longitudinalement entre l'extrémité distale de la tige, ou une zone de cette tige proche de cette extrémité distale, et l'extrémité proximale de la tige, ou une zone proche de cette extrémité proximale. Ce barreau peut être rectiligne entre ces extrémités ou ces zones, ou peut être courbe au niveau de sa portion proximale de façon à suivre une forme courbe au niveau proximal que peut présenter la tige médullaire.

Ladite structure interne pourrait également, selon un exemple non couvert par l'invention, être sous la forme d'une portion proximale pleine reliée à une portion longitudinale s'étendant le long du bord interne de la tige. Cette portion longitudinale peut être à section transversale constante peut présenter une section transversale allant en se réduisant dans la direction distale de sorte que la portion longitudinale présente une forme effilée vue dans le plan frontal.

L'invention sera bien comprise, et d'autres caractéristiques et avantages de celle-ci apparaîtront, en référence au dessin schématique annexé ; ce dessin représente, à titre d'exemples non limitatifs, plusieurs formes de réalisation possibles de l'endoprothèse concernée. Dans ce dessin :
[Fig. 1] est une vue de l'implant huméral d'une prothèse d'épaule, dans le plan frontal, selon une première forme de réalisation ; trois composants de cet implant étant représentés en éclaté ;
[Fig. 2] est une vue de l'implant similaire à la figure 1, les trois composants étant dans un état d'assemblage ;
[Fig. 3] est une vue de l'implant dans le plan sagittal, par le côté externe de cet implant ;
[Fig. 4] est une vue d'une tige médullaire que comprend l'implant, en coupe selon le plan IV-IV de la figure 2 ;
[Fig. 5] est une vue de la tige médullaire en coupe selon le plan V-V de la figure 2 ;
[Fig. 6] est une vue de la tige médullaire de l'implant selon une deuxième forme de réalisation, dans le plan sagittal, par le côté interne de cette tige ;
[Fig. 7] est une vue de la tige en coupe selon le plan VII-VII de la figure 6 ;
[Fig. 8] est une vue de la tige similaire à la figure 7, selon l'invention; et
[Fig. 9] une vue de la tige similaire à la figure 7, selon un exemple non couvert par l'invention.

### Description détaillée

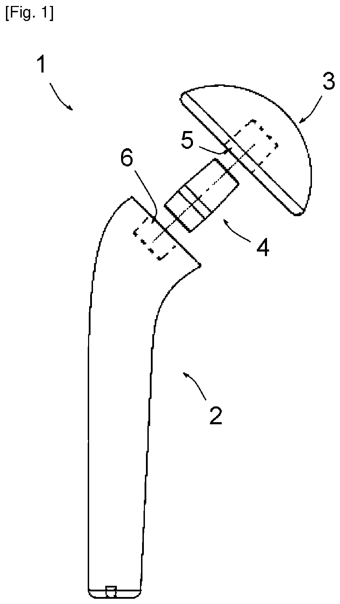

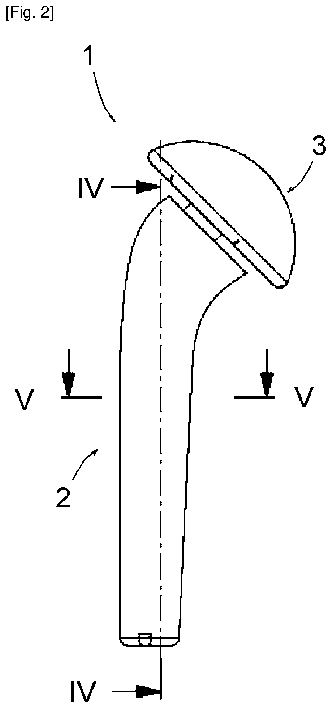

Les figures 1 à 3 représentent l'implant huméral 1 d'une endoprothèse d'articulation de l'épaule, comprenant une tige médullaire 2, une tête d'articulation 3 et un col 4 de jonction de cette tête à cette tige. L'endoprothèse comprend également un implant de glène destiné à être mis en place sur l'omoplate du patient, non décrit car ne faisant pas en lui-même partie de l'invention.

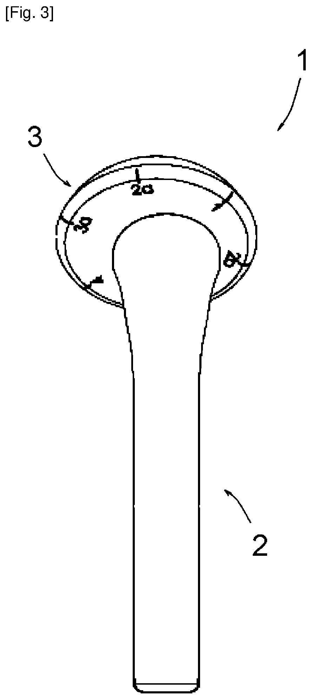

La tête d'articulation 3 est, dans l'exemple représenté, de type dit "anatomique", c'est-à-dire formant une surface articulaire convexe, apte à coopérer avec une surface articulaire conjuguée concave formée par l'implant de glène. Cette tête est de type classique, notamment en matériau métallique, et comprend une cavité 5 légèrement conique de réception avec coincement d'un embout légèrement conique correspondant formé par le col 4. Également dans l'exemple représenté, et comme visible sur la figure 3, la cavité 5 est décentrée par rapport à la tête 3 de façon à permettre de faire varier la position de la tête 3 par rapport à la tige 2 selon la position angulaire de mise en place de cette tête sur l'embout 4, ceci pour permettre d'obtenir une adaptation optimale de l'implant huméral 1 à l'anatomie spécifique du patient. Pour permettre un repérage de la position angulaire de la tête 3 par rapport à la tige 2, la face de cette tête tournée vers cette tige est graduée au niveau de la périphérie de cette face.

Le col 4 est de type classique, notamment en matériau métallique ; outre l'embout précité d'assemblage à la tête 3, il comprend à l'opposé de ce premier embout un embout homologue pour son assemblage à la tige 2, cette dernière comprenant une cavité 6 de forme adaptée à recevoir ce deuxième embout avec coincement.

La tige médullaire 2 présente une forme adaptée à une insertion dans le canal médullaire d'un humérus, après coupe de la partie proximale de cet humérus et alésage approprié du canal médullaire de l'os.

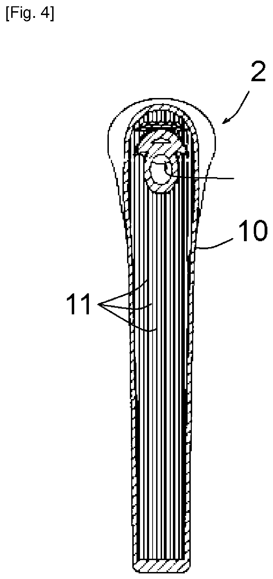

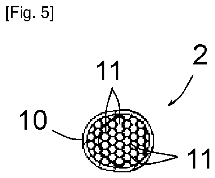

Comme visible sur les figures 4 et 5, la tige 2 présente une paroi d'enveloppe 10, définissant la forme extérieure que présente cette tige, et une pluralité de cloisons 11 définissant une structure en nids d'abeilles, ces cloisons 11 remplissant l'intégralité de l'espace creux délimité intérieurement par cette paroi 10, à l'exception d'une partie pleine proximale délimitant la cavité 6.

Les cloisons 11 forment corps avec la paroi 10, l'ensemble de la tige 2 étant réalisé par un procédé de fabrication dit "d'impression 3D" ou "additif", connu en lui-même, par lequel l'objet à fabriquer est réalisé par la constitution de couches de matériau durcissable successives ; ce matériau est durci en des emplacements spécifiques de chaque couche, destinés à constituer une couche de l'objet à obtenir, et est en particulier polymérisé au moyen d'un laser.

Certaines des cloisons 11 qui délimitent les alvéoles en nids d'abeilles sont orientées selon une direction interne-externe de la tige 2, c'est-à-dire selon une direction gauche-droite sur la figure 5 tandis que les autres cloisons 11 sont orientées selon une direction antérieure-postérieure, c'est-à-dire selon une direction haut-bas sur la figure 5. Les axes longitudinaux des alvéoles délimitées par les cloisons 11 sont par ailleurs, dans l'exemple de réalisation représenté, parallèles à l'axe longitudinal de la tige médullaire 2.

Il sera compris que l'expression "direction inter-externe" désigne une direction allant du bord de cette tige médullaire destiné à se trouver après implantation le long du bord interne de l'humérus au bord opposé destiné à se trouver après implantation le long du bord externe de l'humérus ; de façon similaire, l'expression "direction antérieure-postérieure" désigne la direction allant du bord de la tige 2 destiné à se trouver après implantation le long du bord antérieur de l'humérus au bord de cette tige se trouvant après implantation le long du bord postérieur de l'humérus.

La tige médullaire 2 présente ainsi une structure à paroi d'enveloppe 10 et à pluralité de cloisons 11 ayant des orientations interne-externe et antérieure-postérieure, espacées les unes des autres et formant corps avec ladite paroi d'enveloppe 10. Cette structure s'avère particulièrement intéressante en ce sens qu'elle est apte à supporter la charge s'exerçant sur la tige médullaire 2, du fait de la pluralité de cloisons 11 et du fait que ces cloisons forment corps avec la paroi d'enveloppe 10, tout en présentant une légère capacité de déformation, proche de celle d'un humérus.

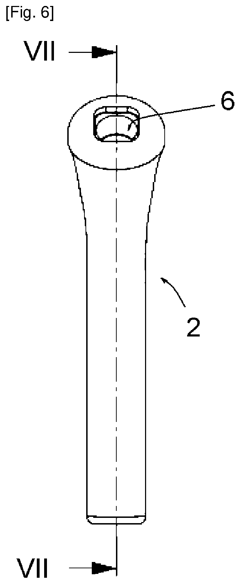

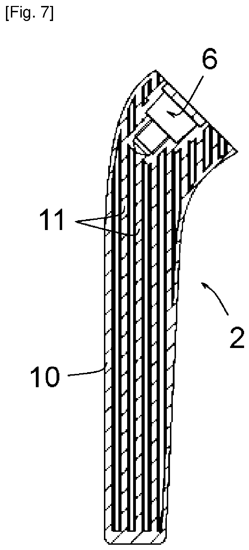

Les figures 6 et 7 représentent une tige médullaire 2 selon une deuxième forme de réalisation, dans laquelle lesdites cloisons 11 internes-externes et antérieures-postérieures ne présentent pas des ondulations comme celles délimitant les alvéoles selon la première forme de réalisation, mais sont au contraire planes et s'entrecroisent à angle droit les unes des autres, la structure interne formée par ces cloisons étant ainsi "en croisillons".

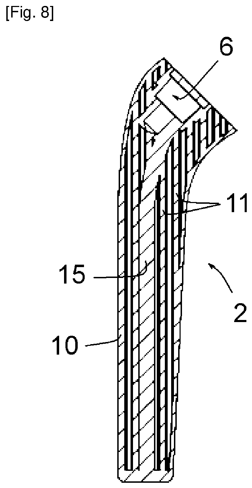

La figure 8 représente une tige médullaire 2 selon l'invention, dans laquelle la tige 2 présente un barreau interne plein 15, s'étendant entre la paroi d'extrémité distale de la tige 2 et au moins une paroi délimitant la cavité 6. Ce barreau 15 forme corps avec la paroi d'enveloppe 10 et les cloisons 11 et est réalisé dans le même matériau que ces parois 10 et cloisons 11 ; il peut avoir toute section transversale appropriée, notamment une section circulaire ; sa dimension selon la direction interne-externe peut notamment représenter, comme visible sur la figure 8, environ le tiers de la dimension interne-externe que présente la tige médullaire au niveau de sa portion distale effilée.

Le barreau 15 permet un renforcement longitudinal localisé de la tige 2.

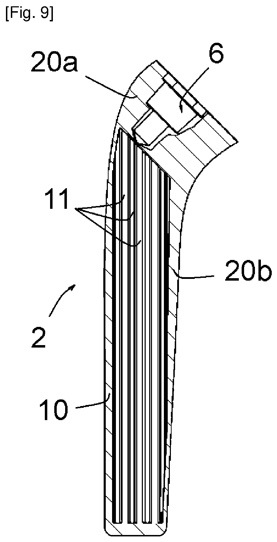

La figure 9 représente une tige médullaire 2 selon un exemple non couvert par l'invention, dans laquelle ledit espace creux occupé par les cloisons 11 ne s'étend pas sur l'intégralité du volume délimité par la paroi d'enveloppe 10. En effet, la tige 2 présente une partie massive, faite dans le même matériau que la paroi 10 et les cloisons 11, située le long d'au moins un bord de la paroi d'enveloppe 10. Dans l'exemple représenté, cette partie massive comprend une portion 20a s'étendant sur une partie proximale de la tige, autour de la cavité 6 et une portion 20b s'étendant sur une partie proximale du bord longitudinal de la tige 2 destiné à se trouver après implantation le long du bord interne de l'humérus, cette portion 20b allant en se réduisant en section transversale dans la direction distale de sorte que la portion 20 présente, vue dans le plan frontal comme selon la figure 9, une forme effilée.

Cette partie pleine, à l'instar du barreau 15, permet également un renforcement longitudinal localisé de la tige 2.

## Revendications

1. Tige médullaire (2) d'endoprothèse d'articulation, destinée à être mise en place dans le canal médullaire d'un os long que comprend l'articulation concernée, comprenant :
- une paroi d'enveloppe (10), définissant la forme extérieure que présente la tige médullaire (2), et délimitant intérieurement un espace creux ; et
- une pluralité de premières cloisons (11), généralement parallèles entre elles, s'étendant entre le côté interne de cette tige médullaire (2), c'est-à-dire le côté destiné à se trouver après implantation le long du bord interne de l'os long, et le côté externe de cette tige médullaire (2), c'est-à-dire le côté destiné à se trouver après implantation le long du bord externe de l'os long, chacune de ces premières cloisons (11) formant corps avec la paroi d'enveloppe (10) au niveau de ces côtés interne et externe de la tige médullaire,
- une structure interne longitudinale (15 ; 20a, 20b) de renforcement longitudinal de la tige médullaire, à section transversale pleine, formant corps avec la paroi d'enveloppe (10) et lesdites premières cloisons (11) et des deuxièmes cloisons (11) lorsque ces deuxièmes cloisons sont présentes,
**caractérisée en ce que** ladite structure interne est sous la forme d'un barreau interne central (15) s'étendant longitudinalement entre une extrémité distale de la tige (2), ou une zone de cette tige proche de cette extrémité distale, et une extrémité proximale de la tige (2), ou une zone proche de cette extrémité proximale.

2. Tige médullaire (2) selon la revendication 1, **caractérisée en ce que** la paroi d'enveloppe (10) a une épaisseur allant de 0,5 à 2 mm.

3. Tige médullaire (2) selon la revendication 1 ou la revendication 2, **caractérisée en ce que** chaque cloison (11) a une épaisseur allant de 0,5 à 1,5 mm.

4. Tige médullaire (2) selon l'une des revendications 1 à 3, **caractérisée en ce que** l'espace entre les cloisons (11) va de 1 à 2 mm.

5. Tige médullaire (2) selon l'une des revendications 1 à 4, **caractérisée en ce que** le nombre de cloisons (11) va de quatre à six cloisons (11) sur une tige médullaire (2) destinée à un patient de petite taille et de six à dix cloisons (11) sur une tige médullaire (2) destinée à patient de grande taille.

6. Tige médullaire (2) selon l'une des revendications 1 à 5, **caractérisée en ce que** les cloisons (11) sont planes.

7. Tige médullaire (2) selon l'une des revendications 1 à 5, **caractérisée en ce que** les cloisons (11) présentent un profil ondulé.

8. Tige médullaire (2) selon l'une des revendications 1 à 7, **caractérisée en ce qu'**elle comprend une pluralité de deuxièmes cloisons (11), généralement parallèles entre elles, ayant une orientation différente de celle desdites premières cloisons (11) et venant ainsi croiser ces premières cloisons, chacune de ces deuxièmes cloisons (11) formant corps avec la paroi d'enveloppe (10) de la tige médullaire (2) et formant corps avec lesdites premières cloisons (2).

9. Tige médullaire (2) selon la revendication 8, caractérisée en ce lesdites premières cloisons (11) et deuxièmes cloisons (11) délimitent entre elles des alvéoles ayant une structure en nid d'abeilles.

## Patentansprüche

1. Medullärer Stab (2) einer Gelenkendoprothese, dazu bestimmt, in dem medullären Kanal eines Röhrenknochens, den das betroffene Gelenk umfasst, eingesetzt zu werden, umfassend:
- eine Mantelwand (10), die die äußerer Form definiert, die der medulläre Stab (2) aufweist, und die im Inneren einen hohlen Raum begrenzt; und
- mehrere erste Trennwände (11), die allgemein parallel zueinander sind und sich zwischen der Innenseite dieses medullären Stabs (2), das heißt der Seite, die dazu bestimmt ist, sich nach Implantation entlang der Innenkante des Röhrenknochens zu befinden, und der Außenseite dieses medullären Stabs (2), das heißt der Seite, die dazu bestimmt ist, sich nach Implantation entlang der Außenkante des Röhrenknochens zu befinden, wobei jede dieser ersten Trennwände (11) mit der Mantelwand (10) im Bereich dieser Innen- und Außenseite des medullären Stabs integral ausgebildet ist,
- eine längsgerichtete innere Struktur (15; 20a, 20b) zur längsgerichteten Verstärkung des medullären Stabs, mit massivem Querschnitt, die mit der Mantelwand (10) und den ersten Trennwänden (11) und zweiten Trennwänden (11) integral ausgebildet ist, wenn diese zweiten Trennwände (11) vorhanden sind,
**dadurch gekennzeichnet, dass** die innere Struktur die Form einer mittigen inneren Stange (15) aufweist, die sich längsgerichtet erstreckt zwischen einem distalen Ende des Stabs (2),
oder einer Zone dieses Stabs in der Nähe von diesem distalen Ende, und einem proximalen Ende des Stabs (2), oder einer Zone in der Nähe von diesem proximalen Ende.

2. Medullärer Stab (2) nach Anspruch 1, **dadurch gekennzeichnet, dass** die Mantelwand (10) eine Dicke von 0,5 bis 2 mm hat.

3. Medullärer Stab (2) nach Anspruch 1 oder Anspruch 2, **dadurch gekennzeichnet, dass** jede Trennwand (11) eine Dicke von 0,5 bis 1,5 mm hat.

4. Medullärer Stab (2) nach einem der Ansprüche 1 bis 3, **dadurch gekennzeichnet, dass** der Raum zwischen den Trennwänden (11) von 1 bis 2 mm beträgt.

5. Medullärer Stab (2) nach einem der Ansprüche 1 bis 4, **dadurch gekennzeichnet, dass** die Anzahl an Trennwänden (11) von vier bis sechs Trennwänden (11) auf einem medullären Stab (2), der für einen kleinen Patienten bestimmt ist, und von sechs bis zehn Trennwänden (11) auf einem medullären Stab (2), der für einen großen Patienten bestimmt ist, beträgt.

6. Medullärer Stab (2) nach einem der Ansprüche 1 bis 5, **dadurch gekennzeichnet, dass** die Trennwände (11) flächig sind.

7. Medullärer Stab (2) nach einem der Ansprüche 1 bis 5, **dadurch gekennzeichnet, dass** die Trennwände (11) ein welliges Profil aufweisen.

8. Medullärer Stab (2) nach einem der Ansprüche 1 bis 7, **dadurch gekennzeichnet, dass** er mehrere zweite Trennwände (11) umfasst, die allgemein parallel zueinander sind und eine Ausrichtung aufweisen, die von der der ersten Trennwände (11) verschieden ist, und dadurch diese ersten Trennwände kreuzen, wobei jede dieser zweiten Trennwände (11) mit der Mantelwand (10 des medullären Stabs (2) integral ausgebildet ist und mit den ersten Trennwänden (11) integral ausgebildet ist.

9. Medullärer Stab (2) nach Anspruch 8, **dadurch gekennzeichnet, dass** die ersten Trennwände (11) und zweiten Trennwände (11) zwischen einander Zellen mit einer Wabenstruktur begrenzen.

## Claims

1. Medullary stem (2) for an endoprosthesis for a joint, intended to be inserted into the medullary canal of a long bone comprising the joint concerned, comprising:
- a casing wall (10), defining the external shape of the medullary stem (2) and delimiting a hollow space internally; and
- a plurality of first partitions (11), generally parallel to each other, extending between the inner side of this medullary stem (2), i.e. the side intended to be located after implantation along the inner edge of the long bone, and the outer side of the medullary stem (2), i.e. the side intended to be located after implantation along the outer edge of the long bone, each of these first partitions (11) forming a single piece with the casing wall (10) at these inner and outer sides of the medullary shaft,
- a longitudinal internal structure (15; 20a, 20b) for longitudinal reinforcement of the medullary stem, with a solid cross-section, forming a single piece with the casing wall (10) and the said first partitions (11) and second partitions (11) when these second partitions are present,
**characterized in that** said internal structure is in the form of a central internal bar (15) extending longitudinally between a distal end of the stem (2), or an area of said stem close to said distal end, and a proximal end of the stem (2), or an area close to said proximal end.

2. Medullary stem (2) according to claim 1, **characterized in that** the casing wall (10) has a thickness ranging from 0.5 to 2 mm.

3. Medullary stem (2) according to claim 1 or claim 2, **characterized in that** each partition (11) has a thickness of 0.5 to 1.5 mm.

4. Medullary stem (2) according to one of claims 1 to 3, **characterized in that** the space between the partitions (11) is between 1 and 2 mm.

5. Medullary stem (2) according to one of claims 1 to 4, **characterized in that** the number of partitions (11) ranges from four to six partitions (11) on a medullary rod (2) intended for a small patient and from six to ten partitions (11) on a medullary rod (2) intended for a large patient.

6. Medullary stem (2) according to one of claims 1 to 5, **characterized in that** the partitions (11) are flat.

7. Medullary stem (2) according to one of claims 1 to 5, **characterized in that** the partitions (11) have a wavy profile.

8. Medullary stem (2) according to one of claims 1 to 7, **characterized in that** it comprises a plurality of second partitions (11), generally parallel to each other, having a different orientation from that of the first partitions (11) and thus intersecting these first partitions, each of these second partitions (11) forming a single piece with the casing wall (10) of the medullary stem (2) and forming a single piece with the said first partitions (2).

9. Medullary stem (2) according to claim 8, **characterized in that** the first partitions (11) and second partitions (11) delimit between them cells having a honeycomb structure.
